(19) Europäisches Patentamt
European Patent Office
Office européen des brevets

(11) Veröffentlichungsnummer: **0 190 568 B1**

# (12) EUROPÄISCHE PATENTSCHRIFT

(45) Veröffentlichungstag der Patentschrift: **27.09.89**

(51) Int. Cl.[4]: **C 07 D 319/08**, C 07 D 319/06, C 07 D 493/10, C 07 D 321/06, C 07 D 317/34, C 07 D 317/72

(21) Anmeldenummer: **86100351.5**

(22) Anmeldetag: **13.01.86**

## (54) Verfahren zur Herstellung von Spiroorthocarbonaten.

**Teilanmeldung 89100251.1 eingereicht am 13/01/86.**

(30) Priorität: **23.01.85 DE 3502106**

(43) Veröffentlichungstag der Anmeldung: **13.08.86 Patentblatt 86/33**

(45) Bekanntmachung des Hinweises auf die Patenterteilung: **27.09.89 Patentblatt 89/39**

(84) Benannte Vertragsstaaten: **CH DE FR GB IT LI**

(56) Entgegenhaltungen:
**DE-A- 3 225 818**
**DE-A- 3 406 049**
**DE-B- 2 407 863**
**US-A- 3 966 768**

**Houben-Weyl, Methoden der organischen Chemie, E4, 699-703 (1983)**
**Justus Liebigs Ann. Chem. 675, 142-143, 1964**

(73) Patentinhaber: **BAYER AG, D-5090 Leverkusen 1 Bayerwerk (DE)**

(72) Erfinder: **Mues, Peter, Dr., Weihers Hecke 30, D-4100 Duisburg 46 (DE)**
Erfinder: **Buysch, Hans-Josef, Dr., Brandenburger Strasse 28, D-4150 Krefeld (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Obwohl cyclische, aliphatische Orthokohlensäureester, insbesondere aliphatische Spiroorthocarbonate, eine interessante Stoffklasse darstellen, sind bisher nur fünf Herstellungsmethoden für die Spiroorthocarbonate bekannt geworden: Umsetzung von Di-thallium(I)-glykolaten mit $CH_2$ unter Abspaltung von Thalliumsulfid (J. Org. Chem. 37, 4198 (1972) ); Reaktion von O,O'-Bis-[tributylstannyl]-Derivaten von Glykolen mit $CS_2$ unter Abspaltung von Bis(tributylzinn)sulfid (J. Org. Chem. 35, 2347 (1970) ); Umsetzung von Alkandiyldioxydibutylstannanen mit $CS_2$ (J. Org. Chem. 36, 1176 (1971) ); Umsetzung von Orthokohlensäureestern mit Diolen unter Abspaltung von Alkohol (DE-OS 3 225 818; Synthesis 1984, 837) und Umsetzung von Epoxiden mit cyclischen fünf- oder sechsgliedrigen Carbonaten in Anwesenheit von Bortrifluoridetherat (DE-OS 3 406 049).

Die zuerst genannten drei Verfahren verlaufen zwar mit recht guten Ausbeuten, jedoch muss mit teuren Metallorganika und toxischem $CS_2$ gearbeitet werden. Bei den letztgenannten Verfahren wird das teure Tetraalkoxymethan als Ausgangsmaterial verwendet, und die Abtrennung des Produkts vom neutralisierten Katalysator ist nicht unproblematisch und anscheinend Ausbeute-bestimmend; die katalysierte Addition von Epoxiden an das Carbonat verläuft nur in sehr schlechten Ausbeuten.

Die Herstellung von Spiroorthocarbonaten gelingt auch durch Umesterung von Orthokohlensäure-tetraestern unter katalytischer Wirkung von p-Toluolsulfonsäure, allerdings mit nicht sehr guter Ausbeute (s. Honben-Weyl, Methoden der organischen Chemie, E 4, S. 699 bis 702, 1983).

Die Verwendung eines sauren Katalysators wie p-Toluolsulfonsäure wird auch in der bereits zitierten DE-OS 32 25 818, Seite 9 als bevorzugte Ausführungsform erwähnt.

Demgegenüber war es überraschend, dass das erfindungsgemässe Verfahren in Gegenwart N-haltiger Basen, die den Stickstoff als Teil eines aromatischen Systems enthalten, in besseren Ausbeuten gelingt.

Gegenstand der vorliegenden Erfindung ist somit ein Verfahren zur Herstellung von aliphatischen Spiroorthocarbonaten der Formel (I)

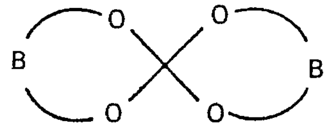

(I),

worin

B für $-\overset{R^3}{\overset{|}{C}}H-\overset{R^4}{\overset{|}{C}}H-$, $-CH_2-C(R^3)(R^4)-CH_2$, $-\overset{R^3}{\overset{|}{C}}H-CH_2-\overset{R^4}{\overset{|}{C}}H-$ oder $-CH_2-C(=CH_2)-CH_2-$ steht und

$R^3$ und $R^4$ gleich oder verschieden sein können und Wasserstoff, $C_1-C_4$-Alkyl oder Phenyl bedeuten oder gemeinsam für $-(CH_2)_m-$ mit m=2, 3, 4, 5 stehen, das dadurch gekennzeichnet ist, dass man Dichlormethanderivate der Formel (II)

$$(R^5O)_2CCl_2 \qquad (II),$$

worin

$R^5$ einen Phenylrest, der unsubstituiert oder gegebenenfalls durch Chlor, Brom, eine Nitro-, Trifluormethyl-, Alkoxygruppe mit 1 bis 4 C-Atomen oder eine Carbalkoxygruppe mit 1 bis 4 C-Atomen ein- oder mehrfach substituiert ist, darstellt, mit aliphatischen Diolen der Formel (III)

$$HO-B-OH \qquad (III),$$

worin

B die obengenannte Bedeutung hat, in Gegenwart stöchiometrischer Mengen N-haltiger Basen, die den Stickstoff als Teil eines aromatischen Systems enthalten, umsetzt.

Als Verbindungen der allgemeinen Formel (II) seien beispielsweise genannt: an den Phenylresten substituierte Diphenyloxymethandichloride, wie Bis-(2,5-dichlorphenoxy)-methandichlorid, Bis-(4-chlorphenoxy)-methandichlorid, Bis-(4-nitrophenoxy)-methandichlorid, Bis-(3-nitrophenoxy)-methandichlorid, Bis-(3-methylphenoxy)-methandichlorid und Bis-(4-methylphenoxy)-methandichlorid und der unsubstituierte Grundkörper, das Diphenoxymethandichlorid. Besonders bevorzugt wird das Diphenoxymethandichlorid eingesetzt (II, $R^5 = C_6H_5$).

Als aliphatische Diole der Formel (III) seien beispielsweise genannt: Glykol, Propandiol-1,2, Butandiol-2,3, Propandiol-1,3, 2-Phenylpropandiol-1,3, 1,1-Cyclohexandimethanol, 2,2-Dimethylpropandiol-1,3, Butandiol-1,3 und Pentandiol-2,4; besonders bevorzugt sind Glykol, Propandiol-1,2, Propandiol-1,3, 2-Methylenpropandiol-1,3 und 2,2-Dimethylpropandiol-1,3. Die Diole werden in stöchiometrischen Mengen oder im Überschuss (etwa 10 bis 100%iger Überschuss) eingesetzt; bevorzugt ist die Verwendung in stöchiometrischen Mengen.

Bei der erfindungsgemässen Herstellung wird die bei der Umsetzung mit dem Dichlormethanderivat (II) entstehende Salzsäure mit N-haltigen Basen gebunden, die den Stickstoff als Teil eines aromatischen Systems enthalten. Beispielsweise können hierzu verwendet werden: Pyridin, das gegebenenfalls auch ein- oder mehrfach durch Chlor, Brom, eine Nitro-, Trifluormethyl-, Alkoxygruppe mit 1 bis 4 Kohlenstoffatomen oder eine Carbalkoxygruppe mit 1 bis 4 Kohlenstoffatomen substituiert sein kann, Chinolin, Isochinolin, Chinazolin, Pyrimidin und N-Alkylimidazol, wie N-Methylimidazol. Die N-haltigen Basen werden in stöchiometrischen Mengen oder im Überschuss (etwa 10 bis 100%iger Überschuss) eingesetzt, bevorzugt ist ein Einsatz von stöchiometrischen Mengen.

Die erfindungsgemässe Umsetzung von Di-

chlormethanderivaten der allgemeinen Formel (II) mit den Diolen der allgemeinen Formel (III) wird bevorzugt in inerten Lösungsmitteln durchgeführt. Geeignete Lösungsmittel hierfür sind beispielsweise Aromaten, wie Toluol und/oder Xylol, halogenierte Kohlenwasserstoffe, wie Methylenchlorid, Ethylenchlorid und/oder Chlorbenzol oder Ether, wie Diethylether, Tetrahydrofuran und/oder Anisol.

Das erfindungsgemässe Verfahren wird im allgemeinen bei Temperaturen von etwa 0 bis 60 °C, bevorzugt 10 bis 40 °C, durchgeführt.

Die Aufarbeitung des Reaktionsgemisches nach dem erfindungsgemässen Verfahren kann beispielsweise so erfolgen, dass das entstandene Hydrochlorid (sofern es im gewählten Lösungsmittel unlöslich ist) abfiltriert oder wässrig extraktiv entfernt wird. Das entstandene Phenol kann extraktiv durch Schütteln mit halbkonzentrierter oder verdünnter wässriger Alkalimetallhydroxid-Lösung oder destillativ nach Entfernung des verwendeten Lösungsmittels abgetrennt werden.

Der nach Entfernung des Hydrochlorids, Phenols und Lösungsmittels verbleibende Rückstand enthält das gewünschte Produkt; dieses kann entweder destillativ oder durch Kristallisation aus einem geeigneten inerten Lösungsmittel isoliert werden, Geeignete inerte Lösungsmittel sind beispielsweise Ester wie Ethylacetat oder Alkohole wie Methanol und/oder Ethanol.

Beispiele 1 bis 5
(allgemeine Herstellungsvorschrift für Spiroorthocarbonate der Formel (I) )

0,1 Mol Dichlordiphenoxymethan (II, $R^5 = C_6H_5$) wurden in 50 ml Dichlormethan gelöst und unter Rühren in ein Gemisch von 0,2 Mol Diol der allgemeinen Formel (V) und 0,2 Mol Pyridin in 50 ml Dichlormethan bei 20 bis 25 °C (Kühlung mit Eiswasser) eingetropft. Nach einstündigem Rühren bei 20 °C wurde die organische Phase nacheinander einmal mit 50 ml Wasser, einmal mit 40 ml halbkonzentrierter wässriger Natriumhydroxid-Lösung und zweimal mit je 50 ml Wasser extrahiert. Nach Trocknen über $Na_2SO_4$ wurde das Lösungsmittel im Wasserstrahlvakuum entfernt und der Rückstand aus Ethylacetat umkristallisiert.

1. Produkt: I, B = $-(CH_2)_3-$, Ausbeute: 90%
2. Produkt: I, B = $-CH_2-C(CH_3)_2-CH_2-$, Ausbeute: 90%
3. Produkt: I, B = $-CH_2-\underset{\underset{CH_2}{\|}}{C}-CH_2$ Ausbeute: 85%
4. Produkt: I, B = $-CH_2-C-CH_2$, Ausbeute: 90%

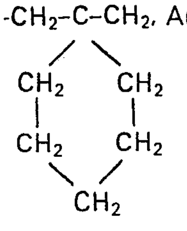

5. Produkt: I, B = $-CH_2-CH_2-$, Ausbeute: 75%

Die Produkte aus den Beispielen 1 bis 5 stimmen in ihren physikalischen Daten mit den in der Literatur angegebenen Daten überein.

## Patentansprüche

1. Verfahren zur Herstellung von aliphatischen Spiroorthocarbonaten der Formel (I)

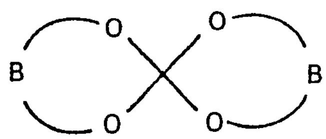

(I),

worin

B für $-\overset{R^3}{CH}-\overset{R^4}{CH}-$, $-CH_2-\overset{R^3\ R^4}{C}-CH_2$, $-\overset{R^3}{CH}-CH_2-\overset{R^4}{CH}-$ oder $-CH_2-\underset{\underset{CH_2}{\|}}{C}-CH_2-$ steht und

$R^3$ und $R^4$ gleich oder verschieden sein können und Wasserstoff, $C_1-C_4$-Alkyl oder Phenyl bedeuten oder gemeinsam für $-(CH_2)_m-$ mit m = 2, 3, 4, 5 stehen, dadurch gekennzeichnet, dass man Dichlormethanderivate der Formel (II)

$(R^5O)_2CCl_2$ (II),

worin

$R^5$ einen Phenylrest, der unsubstituiert oder gegebenenfalls durch Chlor, Brom, eine Nitro-, Trifluormethyl-, Alkoxygruppe mit 1 bis 4 C-Atomen oder eine Carbalkoxygruppe mit 1 bis 4 C-Atomen ein- oder mehrfach substituiert ist, darstellt, mit aliphatischen Diolen der Formel (III)

HO–BO–H (III),

worin

B die obengenannte Bedeutung hat, in Gegenwart stöchiometrischer Mengen N-haltiger Basen, die den Stickstoff als Teil eines aromatischen Systems enthalten, umsetzt.

2. Verfahren nach Anspruch 1, dadurch gekennzeichnet, dass man als N-haltige Basen Pyridin, Chinolin, Isochinolin, Chinazolin, Pyrimidin oder N-Methylimidazol einsetzt.

3. Verfahren nach Ansprüchen 1 und 2, dadurch gekennzeichnet, dass man die Umsetzung bei Temperaturen von 0 bis 60 °C durchführt.

4. Verfahren nach Ansprüchen 1 bis 3, dadurch gekennzeichnet, dass man die Umsetzung in Gegenwart von inerten, organischen Lösungsmitteln durchführt.

5. Verfahren nach Ansprüchen 1 bis 4, dadurch gekennzeichnet, dass man als inerte organische Lösungsmittel Toluol, Xylol, Methylenchlorid, Ethylenchlorid, Chlorbenzol, Diethylether, Tetrahydrofuran und/oder Anisol einsetzt.

## Revendications

1. Procédé de production de spiro-orthocarbonates aliphatiques de formule (I)

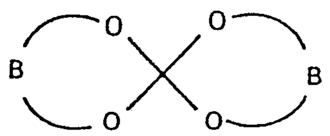

(I),

dans lequel

B représente 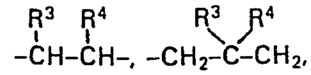

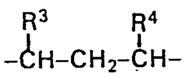 ou 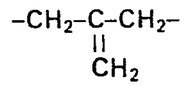 et

$R^3$ et $R^4$ peuvent être identiques ou différents et représentent l'hydrogène, un groupe alkyle en $C_1$ à $C_4$ ou un groupe phényle ou forment conjointement un groupe $-(CH_2)_m-$ dans lequel m a la valeur 2, 3, 4 ou 5, caractérisé en ce qu'on fait réagir des dérivés de dichlorométhane de formule (II)

$(R^5O)_2CCl_2$ (II)

dans laquelle

$R^5$ est un reste phényle qui n'est pas substitué ou qui est substitué le cas échéant une ou plusieurs fois par du chlore, du brome, un groupe nitro, trifluorométhyle, alkoxy ayant 1 à 4 atomes de carbone ou carbalkoxy ayant 1 à 4 atomes de carbone, avec des diols aliphatiques de formule (III)

HO–BO–H (III)

dans laquelle

B a la définition indiquée ci-dessus, en présence de quantités stoechiométriques de bases azotées qui contiennent l'azote comme partie d'un système aromatique.

2. Procédé suivant la revendication 1, caractérisé en ce qu'on utilise comme bases azotées la pyridine, la quinoléine, l'isoquinoléine, la quinazoline, la pyrimidine ou le N-méthylimidazole.

3. Procédé suivant les revendications 1 et 2, caractérisé en ce qu'on conduit la réaction à des températures de 0 à 60 °C.

4. Procédé suivant les revendications 1 à 3, caractérisé en ce qu'on conduit la réaction en présence de solvants organiques inertes.

5. Procédé suivant les revendications 1 à 4, caractérisé en ce qu'on utilise comme solvants organiques inertes le toluène, le xylène, le chlorure de méthylène, le chlorure d'éthylène, le chlorobenzène, l'éther de diéthyle, le tétrahydrofuranne et/ou l'anisole.

## Claims

1. A process for the production of aliphatic spiro-orthocarbonates corresponding to formula (I)

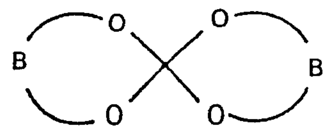

(I),

in which

B represents 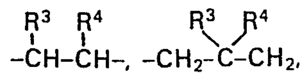

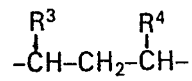 or 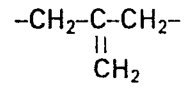 and

$R^3$ and $R^4$ may be the same or different and represent hydrogen, $C_1$–$C_4$ alkyl or phenyl or together represent $-(CH_2)_m-$ with m = 2, 3, 4, 5, characterized in that dichloromethane derivatives corresponding to formula (II)

$(R^5O)_2CCl_2$ (II),

in which

$R^5$ is a phenyl radical which is unsubstituted or optionally substituted by chlorine, bromine, a nitro, trifluoromethyl, alkoxy group containing 1 to 4 C atoms or a carbalkoxy group containing 1 to 4 C atoms either once or several times, are reacted with aliphatic diols corresponding to formula (III)

HO–B–OH (III),

in which

B is as defined above, in the presence of stoichiometric quantities of N-containing bases which contain the nitrogen as part of an aromatic system.

2. A process as claimed in claim 1, characterized in that pyridine, quinoline, isoquinoline, quinazoline, pyrimidine or N-methyl imidazole is used as the N-containing base.

3. A process as claimed in claims 1 and 2, characterized in that the reaction is carried out at temperatures of from 0 to 60 °C.

4. A process as claimed in claims 1 to 3, characterized in that the reaction is carried out in the presence of inert organic solvents.

5. A process as claimed in claims 1 to 4, characterized in that toluene, xylene, methylene chloride, ethylene chloride, chlorobenzene, diethyl ether, tetrahydrofuran and/or anisole is/are used as the inert organic solvent(s).